# EUROPEAN PATENT SPECIFICATION

(11) **EP 2 547 683 B1**
(45) Date of publication and mention of the grant of the patent: **19.02.2014**
(21) Application number: 11718244.4
(22) Date of filing: 15.03.2011
(51) Int. Cl.: C07D 487/04

(54) **METHOD OF PREPARING SITAGLIPTIN**
VERFAHREN ZUR HERSTELLUNG VON SITAGLIPTIN
PROCÉDÉ POUR LA PRÉPARATION DE SITAGLIPTIN

(30) Priority: 16.03.2010 CZ 20100198
(43) Date of publication of application: 23.01.2013
(73) Proprietor: Zentiva, K.S., 102 37 Praha 10 (CZ)
(72) Inventor: RICHTER, Jindrich, 530 02 Pardubice (CZ); JIRMAN, Josef, 100 00 Praha 10 (CZ)
(74) Representative: Jirotkova, Ivana
(86) International application number: PCT/CZ2011/000021
(87) International publication number: WO 2011/113399

(56) References cited:
- WO-A1-97/47632
- DIETRICH STEINHUEBEL, YONGKUI SUN, KAZUHIKO MATSUMURA, NOBORU SAYO AND TAKAO SAITO: "Direct Asymmetric Reductive Amination", JOURNAL OF THE AMERICAN CHEMICAL SOCIETY, vol. 131, 2009, pages 11316-11317, XP002645235, DOI: 10.1021/ja905143m cited in the application

## Description

### Technical Field

The present invention relates to an efficient method of preparing sitagliptin. The method includes enantioselective hydrogenation of prochiral derivative of beta-aminoacrylic acid catalyzed by a complex of Ru with a paracyclophanebisphosphine ligand.

### State of the art

Sitagliptin is (R)-7-(1-oxo-3[(R)-amino]-4-(2,4,5-trifluorophenyl)-butyl)-3-(trifluoromethyl-5,6,7,8-tetrahydro-1,2,4-triazolo[4,3-a]pyrazine represented by formula I.

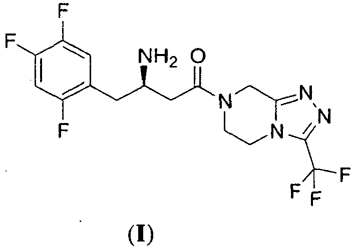

Sitagliptin is an oral inhibitor of the enzyme dipeptidylpeptidase-4 (DPP-IV), which facilitates regulation of metabolism of saccharides in patients with type II diabetes. Sitagliptin can be used in monotherapy or in combination with metformin, or with PPARγ agonists (for instance, thiazolidinediones).

Patent US 6 699 871 describes the class of beta-amino-tetrahydrotriazolo[4,3-a]pyrazines that have an ability to inhibit DPP-IV and can thus be used for treating type II diabetes.

Sitagliptin represented by formula I is prepared by asymmetrical reduction of the enamino-amide precursor represented by formula II. A high enantiomeric excess and, at the same time, a high yield of the asymmetrical reduction of the C=C double bond of the enamino-amide represented by formula II have so far only been reached by means of complex catalysts of Ru with the BINAP and SEGPHOS ligands (J. Am. Chem. Soc. 2009 (131), 11316-11317), or of Rh with ferrocenylbisphosphine ligands of the Josiphos type.

International application WO 2004/085378 describes a process of preparing sitagliptin by asymmetrical hydrogenation of the enamino-amide penultimate of formula II using a complex of Rh with a chiral ferrocenylbisphosphine ligand.

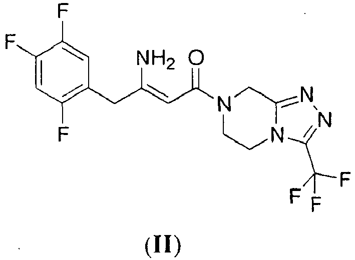

International application WO 2006/081151 extends the above mentioned method of asymmetrical hydrogenation by using complex compounds of transition metals with ferrocenylbisphosphine ligands in the presence of an ammonium salt.

International application WO 2005/097733 describes hydrogenation using complex catalysts of Rh with mono- and bisphosphine ligands.

Verification of known procedures has shown that, in hydrogenations on catalysts so far used for this purpose, reduction of one of fluorine atoms from the aromatic ring always occurs in a non-negligible extent with formation of at least one of three isomers of desfluorositagliptin represented by formulas III, IV and V.

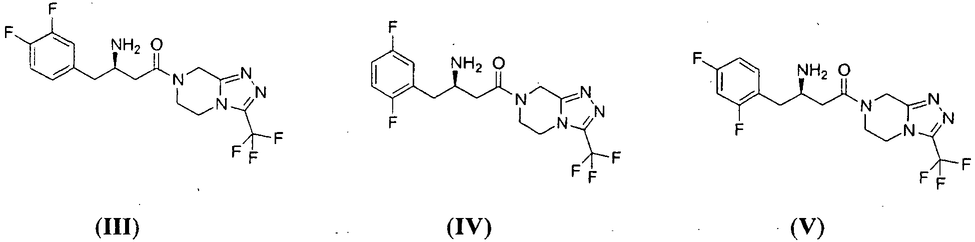

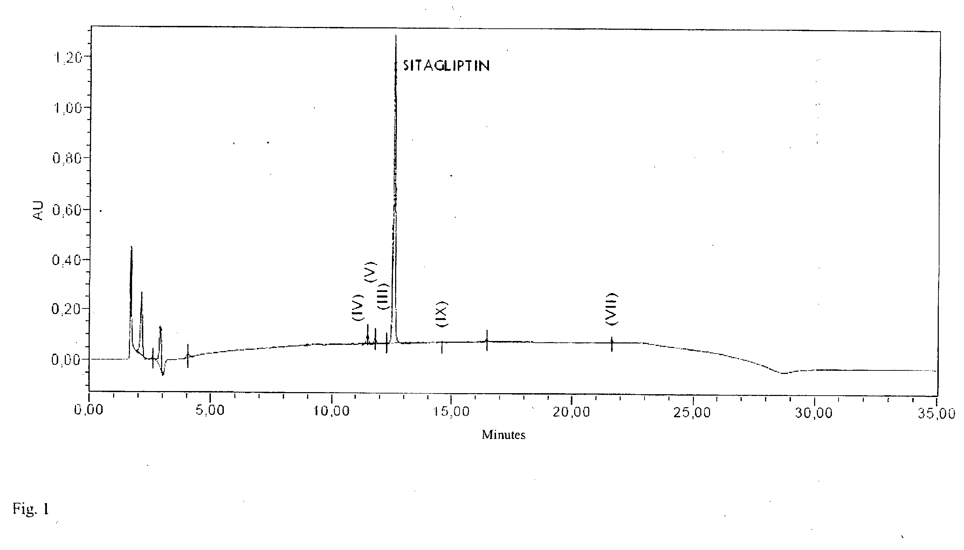

In addition, it has been found that impurities III, IV and V cannot practically be efficiently removed from sitagliptin by usual purification operations, such as crystallization or preparative chromatography in order that the product would have quality required for active pharmaceutical ingredients (API). As shown by the comparative examples, in a product manufactured according to the state of the art, one must count with a content of at least one of impurities III, IV and V in an amount exceeding the limit for a single identified impurity permitted for API by directives of ICH (International Conference for Harmonization). Figure 1 shows how difficult is the chromatographic separation of desfluorositagliptins under conditions of optimized analytical separation. It has been found that with higher column loadings, modelling preparative separation, desfluorositagliptins III, IV and V co-elute with the main peak of sitagliptin.

### Disclosure of invention

This patent application presents an efficient process of preparing sitagliptin of formula I by hydrogenation of the enamine-amide derivative of formula II using a complex formed by reaction of a Ru precursor with the chiral ligand (R) or (S)-pseudo-o-bisphosphino-[2,2]-paracyclophane of general formula VI 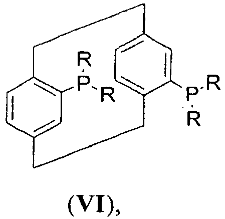
wherein R are same or different substituents from the group consisting of C₁₋₄ alkyls, C₃₋₈ cycloalkyls and Ar,
wherein Ar is an unsubstituted or substituted aryl, which can be, for example, phenyl, 4-methyl-phenyl, 3,5-dimethyl-phenyl, 4-methoxy-phenyl, 4-fluoro-phenyl, or 3,5-bis-(trifluoromethyl)phenyl,
as a catalyst.

Surprisingly, hydrogenation by this method has proved to be highly enantioselective and chemoselective; unexpectedly preferably, it solves the problem of formation of hard to remove impurities. The said process is preferably carried out in presence of an ammonium salt in the reaction mixture.

An advantage of the described process of hydrogenation of the enamino-amide derivative of formula II to the beta-amino acid of formula I using a complex formed by reaction of a Ru precursor with the chiral ligand (R) or (S)-pseudo-o-bisphosphino-[2,2]-paracyclophane of general formula VI, consists in high enantioselectivity (ee higher than 96%) attained in combination with unexpectedly good chemoselectivity so that the process according to this invention successfully solves the problem of undesirable reduction of one of the fluorine atoms from the aromatic ring of compound II with formation of non-removable impurities III, IV and V. In the product according to this invention, these impurities are not present in detectable amounts.

A non-negligible advantage of the said process in comparison with known processes is also high stability of the used complex catalyst as well as respective components from which the desired complex catalyst can be prepared.

The invention also relates to a product obtainable by the above described process, which is characterized by an enantiomeric excess higher than 96% and contents of desfluorositagliptins III, IV and V lower than 0.05% (this is HPLC area %; area % is a ratio of the integrated response of the peak to the integrated response of all peaks detected in the chromatogram, HPLC means high-performance liquid chromatography).

### Detailed description of invention

The present invention relates to a highly efficient process of preparing sitagliptin of formula I. The process is based on hydrogenation of the prochiral enamine-amide of formula II in a suspension or solution in the presence of a complex formed by reaction of a Ru precursor with the chiral ligand (R) or (S)-pseudo-*o*-bisphosphino-[2,2]-paracyclophane of general formula VI, wherein the complex catalyst can be:
a) generated in-situ by simultaneous or successive addition of the Ru precursor and the chiral (R) or (S)-pseudo-*o*-bisphosphino-[2,2]-paracyclophane of general formula VI to the reaction mixture, or
b) prepared separately and (isolated or without isolation) then added to the reaction mixture.

Some ligands of general formula VI are available commercially and known under names such as, for instance, PhanePhos or Xylyl-PhanePhos.

Enantioselectivity of the hydrogenation is given by the used enantiomer of the ligand of general formula VI, the configuration of which determines the configuration of the newly formed chiral centre in the compound of formula I.

Asymmetrical hydrogenation is carried out in a solvent capable of bringing, under conditions of hydrogenation, at least a small part of the introduced catalyst and substrate into a solution. The solvent used can be, in particular:
under the conditions of hydrogenation, liquid non-branched or branched aliphatic, alicyclic or aromatic hydrocarbons, for instance, petroleum ether, hexane, heptane, cyclohexane, methylcyclohexane, benzene, toluene, xylene, ethylbenzene, cumene; ethers, for instance, diethylether, methyl-tert-butylether, diisopropylether, dimethoxyethane, tetrahydrofuran, anisole or dioxan; non-branched or branched aliphatic alcohols, alicyclic alcohols, or arylalkylalcohols, such as for instance, methanol, ethanol, propanol, butanol, amylalcohol, cyclohexanol or benzylalcohol; esters of carboxylic acids with non-branched or branched aliphatic alcohols, for instance, methyl acetate, ethyl acetate, isopropyl acetate or butyl acetate; halo derivatives, such as dichloromethane, dichloroethane, chlorobenzene or freons; lower carboxylic acids, such as acetic acid or propionic acid; their amides, such as formamide, N-methylformamide, N,N-dimethylacetamide or N-methylpyrrolidone; nitriles, such as acetonitrile; or substances with combination of functional groups, such as trifluoroethanol, hexafluoro-2-propanol, methoxyethanol. The solvent can be composed of a single substance or a mixture of substances.

The asymmetrical reduction runs preferably in the presence of an ammonium salt, for instance, ammonium chloride, ammonium phosphate, ammonium formate, ammonium acetate, ammonium oxalate, ammonium carbonate, ammonium salts of salicylic, citric or camphoric acids. The amount of the ammonium salt used ranges between 3 and 1000 mol. % with respect to the prochiral enamine of formula II.

Reaction temperature appropriate for the said hydrogenation process ranges between 10 and 90 °C. Particularly preferred is the temperature range 40 - 70 °C.

The process runs in a hydrogen-containing atmosphere, especially preferably at hydrogen pressure 1 - 4 MPa.

The Ru precursor used for the said process can be, for instance, [Ru^{II}(p-cymene)Cl₂]₂ dimer, bis(2-methylallyl)(1,5-cyclooctadiene)ruthenium^{II}, dichloro(1,5-cyclooctadiene)ruthenium^{II}, dichloro(mesitylene)ruthenium^{II} dimer, bis(η⁵-2,4-dimethylpentadienyl)ruthenium^{II}, bis(cyclopentadienyl)ruthenium^{II}, bis(ethylcyclopentadienyl)ruthenium^{II}, bis(pentamethylcyclopentadienyl)ruthenium^{II} or chloro(pentamethylcyclopentadienyl) (cyclooctadiene)ruthenium^{II}.

The present invention is documented by the following examples. The said examples of embodiments are illustrative only and do not limit the conditions of the embodiments in any way.

### Brief description of specific drawings

**Fig. 1****:** HPLC chart of sitagliptin showing very close retention times of basic impurities desfluorositagliptin (III), (IV) and (V) (RRT = 0.92; 0.94 and 0.98, resp.) under the conditions of analytical high-pressure liquid chromatography.

### Working examples of invention

### Example 1

General method of asymmetrical hydrogenation of β-enamino-amide (II) to sitagliptin (I) (common for the following working examples and comparative examples according to the state of the art)

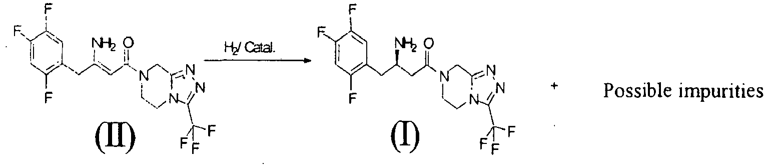

### Possible impurities:

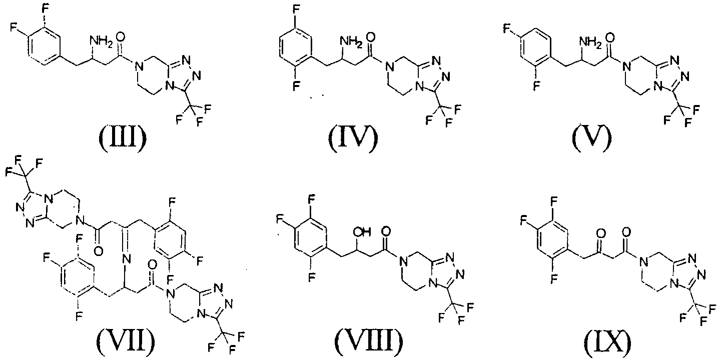

A chiral bisphosphine ligand (24 µmol) was suspended in 10 ml of dry TFE (2,2,2-tritluoroethanol) in a 25 ml reaction vessel. A solution of a Ru or Rh precursor in 3 ml of dry TFE was added dropwise into the stirred suspension at laboratory temperature during 10 minutes. The mixture was stirred for additional 1.5 h under formation of a clear solution. An enamine-amide (II, 1.3 g; 3.2 mmol) and ammonium salicylate (1.9 g; 5.8 mmol) was added to the reaction mixture. The reaction mixture was placed into an autoclave and hydrogenated at 50 °C and pressure of 3 MPa (30 bar) for 24 h. The cooled reaction mixture was concentrated under reduced pressure. The concentrated mixture was dissolved in toluene (13 ml) and extracted with a 0.5 M aqueous solution of phosphoric acid (3 x 6 ml). The separated aqueous layer was alkalized with a 4 M aqueous solution of NaOH to pH 12 and extracted with dichloromethane (2 x 5 ml). After evaporation of the dichloromethane solution, crude product was obtained in the form of a solid foam. Chemical purity of the obtained product was monitored by means of HPLC; optical purity by means of HPLC and capillary electrophoresis (CE).

The following compounds were used as chiral bisphosphine ligands:

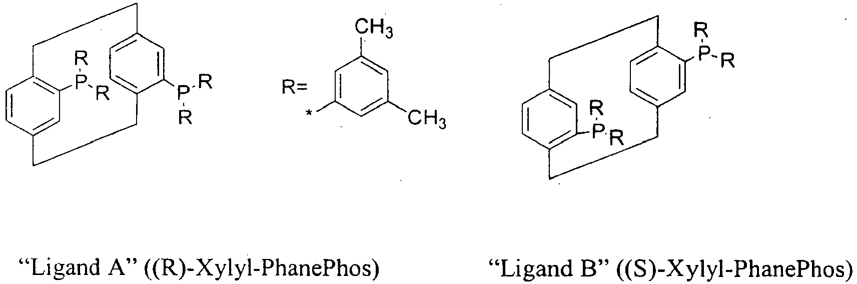

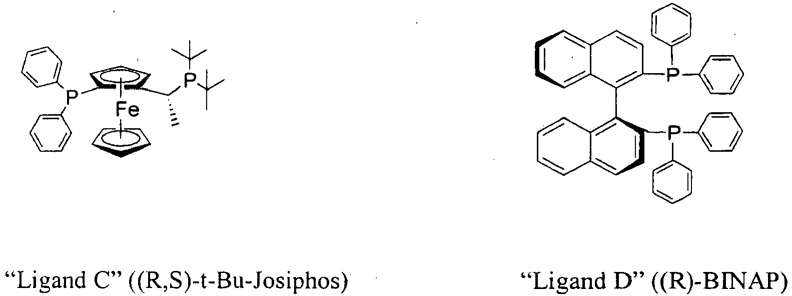

### Example 2 (Ligand A)

According to the above described general procedure, a complex catalyst was prepared by reaction of ligand A with [Ru^{II}(p-cymene)Cl₂]₂. Using the thus obtained catalyst, crude product was obtained by hydrogenation: (R)-sitagliptin of optical purity 97.4% ee and yield 85%; content of sitagliptin 99.1%; content of 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (impurity V) was < 0.05%. The desfluorositagliptins 7-(1-oxo-3[(R)-amino]-4-(3,4-trifluorophenyl)-butyl)-3-(trifluoromethyl-5,6,7,8-tetrahydro-1,2,4-triazolo[4,3-a]pyrazine (III) and 7-(1-oxo-3[(R)-mino]-4-(2,5-trifluorophenyl)-butyl)-3-(trifluoromethyl-5,6,7,8-tetrahydro-1,2,4-triazolo[4,3-a]pyrazine (IV) were not detected.

### Example 3 (Ligand B)

According to the above described general procedure, a complex catalyst was prepared by reaction of ligand B with [Ru^{II}(p-cymene)Cl₂]₂. Using the thus obtained catalyst, crude product was obtained by hydrogenation: (S)-sitagliptin of optical purity 96% ee and yield 98%; content of sitagliptin 92.1%. All the three isomers of desfluorositagliptin 7-(1-oxo-3[(R)-amino]-4-(3,4-trifluorophenyl)-butyl)-3-(trifluoromethyl-5,6,7,8-tetrahydro-1,2,4-triazolo[4,3-a]pyrazine (III), 7-(1-oxo-3[(R)-amino]-4-(2,5-trifluorophenyl)-butyl)-3-(trifluoromethyl-5,6,7,8-tetrahydro-1,2,4-triazolo[4,3-a]pyrazine (IV), 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (V) were under the detection limit of 0.05%.

### Example 4 (Ligand C) (comparative)

According to the above described general procedure, a complex catalyst was prepared by reaction of ligand C with [Rh¹(cod)Cl]₂. Using the thus obtained catalyst, (R)-sitagliptin was obtained by hydrogenation in optical purity of 95.8% ee and yield 78%; content of sitagliptin 94.9%. All the three isomers of desfluorositagliptin 7-(1-oxo-3[(R)-amino]-4-(4,5-trifluorophenyl)-butyl)-3-(trifluoromethyl-5,6,7,8-tetrahydro-1,2,4-triazolo[4,3-a]pyrazine (III), 7-(1-oxo-3[(R)-amino]-4-(2,5-trifluorophenyl)-butyl)-3-(trifluoromethyl-5,6,7,8-tetrahydro-1,2,4-triazolo[4,3-a]pyrazine (IV), 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (V) were identified in the product in a total amount of 3%.

### Example 5 (ligand D) (comparative)

According to the above described general procedure, a complex catalyst was prepared by reaction of ligand D with [Ru^{II}(p-cymene)Cl₂]₂. Using the thus obtained catalyst (R)-sitagliptin was obtained by hydrogenation in optical purity 98.4% ee and yield 86%; the content of the desfluorositagliptin 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (V) was 0.3%.

The obtained crude product was dissolved in hot toluene. After cooling down, colourless crystalline sitagliptin was obtained with 73% yield and content of desfluorositagliptin (V) 0.29%. Repeated crystallization from toluene provided the yield 88% and the product contained 0.29% of desfluorositagliptin (V).

### Example 6

The crude product obtained according to example 5 was further purified by conversion to a salt of organic or inorganic acid, by dissolving in methanol and adding a corresponding amount of the respective acid.
A) sitagliptin (1 g, 2.5 mmol) was dissolved in methanol (30 ml); a solution of orthophosphoric acid (0.17 ml, 2.5 mmol) in methanol (3 ml) was added dropwise to the clear solution at laboratory temperature. The precipitated white salt was filtered and washed with a small amount of methanol. 1 g of sitagliptin*H₃PO₄ was obtained; the content of the desfluorositagliptin 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (V) was 0.3%.
   Sitagliptin*H₃PO₄ was further recrystallized from a mixture of methanol/water (9/1) with 95% yield. The content of the desfluorositagliptin 7-(1-oxo-3[(R)-amino]-4-(2,4-trifluorophenyl)-butyl)-3-(tritluoromethyl-5,6,7,8-tetrahydro-1,2,4-triazolo[4,3-a]pyrazine (V) was 0.28%.
B) sitagliptin (1 g, 2.5 mmol) was dissolved in methanol (30 ml); a solution of O,O'-dibenzoyl-L-tartaric acid (0.96 g, 2.7 mmol) in methanol (3 ml) was added dropwise to the clear solution at laboratory temperature. The precipitated white salt was filtered and washed with a small amount of methanol. 1.6 g of sitagliptin*O,O'-dibenzoyl-L-tartrate was obtained. The content of the desfluorositagliptin 7-(1-oxo-3[(R)-amino]-4-(2,4-trifluorophenyl)-butyl)-3-(tritluoromethyl-5,6,7,8-tetrahydro-1,2,4-triazolo[4,3-a]pyrazine (V) was 0.29%.
   Sitagliptin*O,O'-dibenzoyl-L-tartrate was further recrystallized from a mixture of methanol/water (9/1) with 85% yield. The content of the desfluorositagliptin 7-(1-oxo-3[(R)-amino]-4-(2,4-trifluorophenyl)-butyl)-3-(trifluoromethyl-5,6,7,8-tetrahydro-1,2,4-triazolo[4,3-a]pyrazine (V) was 0.27%. The obtained product was further recrystallized from a mixture of methanol/acetic acid (10/1) with yield 57%. The content of desfluorositagliptin (V) decreased to 0.22%. Further recrystallization from the same mixture of solvents provided a product with 59% yield and the content of desfluorositagliptin (V) 0.18%.

## Claims

1. A method of preparing sitagliptin of formula I 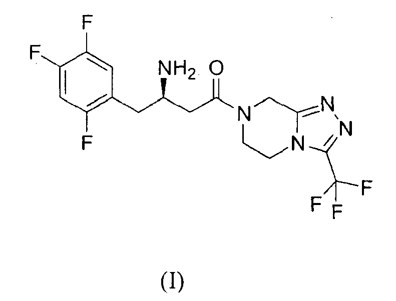 comprising hydrogenation of an enamine-amide precursor of formula II 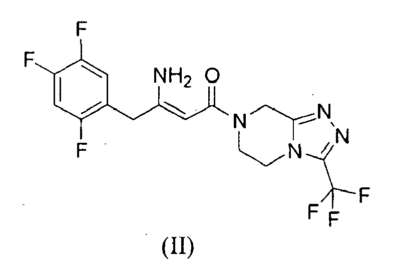 **characterized in that** the hydrogenation is carried out in a suspension or a solution under catalysis with a complex compound formed of Ru and an (R) or (S)-pseudo-*o-*bisphosphino-[2,2]-paracyclophane ligand of general formula VI 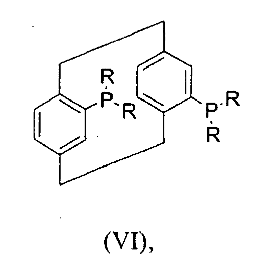
wherein R are the same or different substituents from the group consisting of C₁₋₄ alkyls, C₃₋₈ cycloalkyls and Ar,
and Ar is an unsubstituted or substituted aryl,
and, optionally, other component(s) necessary for formation of the complex compound.

2. The method according to claim 1, **characterized in that** the complex compound serving as the catalyst contains an (R) or (S)-pseudo-*o*-bisphosphino-[2,2]-paracyclophane ligand of general formula VI, wherein R is an unsubstituted or substituted aryl.

3. The method according to claims 1 or 2, **characterized in that** the complex compound serving as the catalyst contains an (R) or (S)-pseudo-*o*-bisphosphino-[2,2]-paracyclophane ligand of formula X

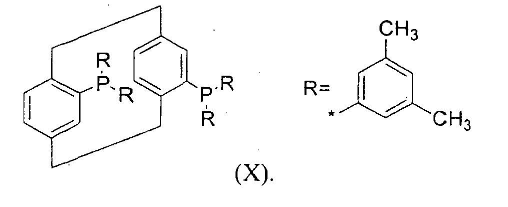

4. The method according to any one of claims 1-3, **characterized in that** the complex compound serving as the catalyst contains an (R)-pseudo-o-bisphosphino-[2,2]-paracyclophane ligand of formula X.

5. The method according to any one of claims 1-4, **characterized in that** the complex compound serving as catalyst is prepared separately or in-situ.

6. The method according to any one of claims 1-5, **characterized in that** it is carried out in an atmosphere containing gaseous hydrogen.

7. The method according to any one of claims 1-6, **characterized in that** it is carried out in the presence of an ammonium salt.

8. The method according to claim 7, **characterized in that** the ammonium salt is chosen from the group including ammonium chloride, ammonium phosphate, ammonium formate, ammonium acetate, ammonium oxalate, ammonium carbonate, ammonium salts of salicylic, citric or camphoric acids.

9. The method according to any one of claims 7 and 8, **characterized in that** the ammonium salt is chosen from the group including the ammonium salt of salicylic acid, the ammonium salt of formic acid and the ammonium salt of camphoric acid.

10. The method according to any one of claims 1-9, **characterized in that** it is carried out in an atmosphere containing hydrogen.

11. The method according to any one of claims 1-10, **characterized in that** it is carried out under a pressure of hydrogen of 1 - 4 MPa.

12. The method according to any one of claims 1-11, **characterized in that** it is carried out at temperature of 10 - 90 °C.

13. The method according to any one of claims 1-12, **characterized in that** it is carried out at temperature of 40 - 70 °C.

14. The method according to any one of claims 1-13, **characterized in that** it is carried out in C1 - C6 alcohols.

15. The method according to any one of claims 1-14, **characterized in that** it is carried out in 2,2,2-trifluoroethanol.

## Patentansprüche

1. Verfahren zur Herstellung von Sitagliptin der Formel I 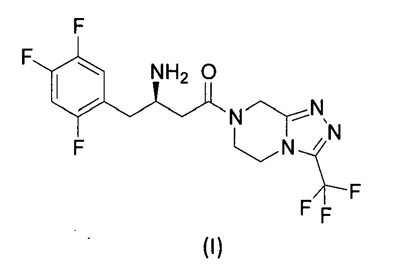 umfassend Hydrogenation von einem Enamin-Amidpräkursor der Formel II 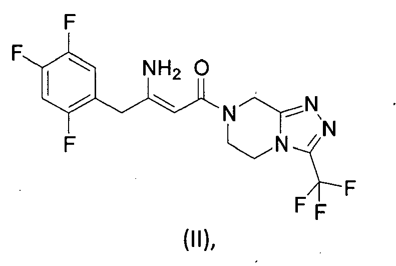 **dadurch gekennzeichnet, dass** die Hydrogenation in einer Suspension oder Lösung mit Katalyse der Komplexverbindung, gebildet aus Ru und einem (R) oder (S)-Pseudo-*o-*bisphosphino-[2,2]-paracyclophan-Ligand der Allgemeinformel VI 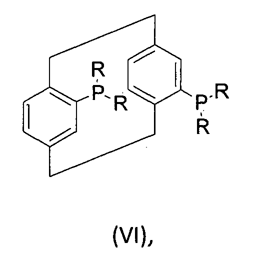
wobei R gleiche oder verschiedene Substituenten der Gruppe bestehend von C₁₋₄, Alkyls, C₃₋₈ Cykloalkyls und Ar sind,
und Ar ein substituiertes oder nicht substituiertes Aryl ist,
und gegebenenfalls anderer (en) Komponente(n) erforderlich für Aufbildung der Komplexverbindung, durchgeführt wird.

2. Verfahren gemäß Anspruch 1, **dadurch gekennzeichnet, dass** die Komplexverbindung, dienend als Katalysator, einen (R) oder (S)-Pseudo-o-bisphosphino-[2,2]-paracyclophan-Ligand der Allgemeinformel VI, worin R ein substituiertes oder nicht substituiertes Aryl ist, enthält.

3. Verfahren gemäß Ansprüche 1 oder 2, **dadurch gekennzeichnet, dass** die Komplexverbindung dienend als Katalysator einen (R) oder (S)-Pseudo-*o*-bisphosphino-[2,2]-paracyclophan-Ligand der Formel X 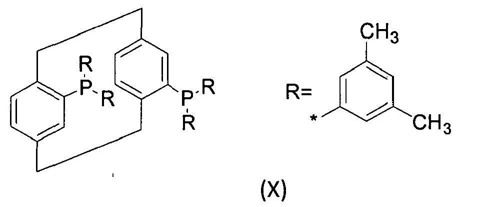 enthält.

4. Verfahren gemäß einem der Ansprüche 1-3, **dadurch gekennzeichnet, dass** die Komplexverbindung dienend als Katalysator einem (R)-Pseudo-o-bisphosphino-[2,2]-paracyclophan-Ligand der Formel X enthält.

5. Verfahren gemäß einem der Ansprüche 1-4, **dadurch gekennzeichnet, dass** die Komplexverbindung dienend als Katalysator separat oder in-situ hergestellt wird.

6. Verfahren gemäß einem der Ansprüche 1-5, **dadurch gekennzeichnet, dass** es in einer Wasserstoffgas enthaltenden Atmosphäre durchgeführt wird.

7. Verfahren gemäß einem der Ansprüche 1-6, **dadurch gekennzeichnet, dass** es in Gegenwart von einem Ammoniumsalz durchgeführt wird.

8. Verfahren gemäß Anspruch 7, **dadurch gekennzeichnet, dass** das Ammoniumsalz aus der Gruppe umfassend Ammoniumchlorid, Ammoniumphosphat, Ammoniumformiat, Ammoniumacetat, Ammoniumoxalat, Ammoniumkarbonat, Ammoniumsalze der Salizylsäure, Zitronensäure, Kampfersäure ausgewählt ist.

9. Verfahren gemäß einem der Ansprüche 7 und 8, **dadurch gekennzeichnet, dass** das Ammoniumsalz aus der Gruppe umfassend das Ammoniumsalz der Salizylsäure, das Ammonium salz der Ameisensäure und das Ammoniumsalz der Kampfersäure ausgewählt ist.

10. Verfahren gemäß einem der Ansprüche 1-9, **dadurch gekennzeichnet, dass** es in einer Wasserstoff enthaltenden Atmosphäre durchgeführt wird.

11. Verfahren gemäß einem der Ansprüche 1-10, **dadurch gekennzeichnet, dass** es unter Wasserstoffdruck von 1-4 MPa durchgeführt wird.

12. Verfahren gemäß einem der Ansprüche 1-11, **dadurch gekennzeichnet, dass** es bei einer Temperatur von 10-90 °C durchgeführt wird.

13. Verfahren gemäß einem der Ansprüche 1-12, **dadurch gekennzeichnet, dass** es bei einer Temperatur von 40-70 °C durchgeführt wird.

14. Verfahren gemäß einem der Ansprüche 1-13, **dadurch gekennzeichnet, dass** es in C1-C6 Alkoholen durchgeführt wird.

15. Verfahren gemäß einem der Ansprüche 1-14, **dadurch gekennzeichnet, dass** es in 2,2,2-Trifluorethanol durchgeführt wird.

## Revendications

1. Procédé pour la préparation de la sitagliptine de formule I 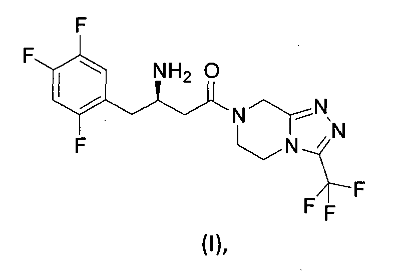 comprenant l'hydrogénation d'un précurseur enamine-amide de formule II 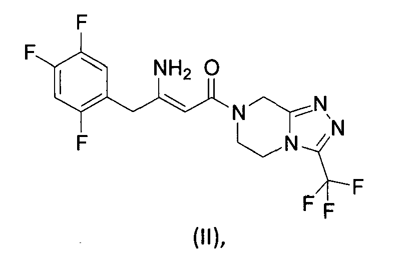 **caractérisé en ce que** l'hydrogénation est effectuée dans une suspension ou solution sous catalyse avec un composé complexe, formé du Ru et un ligand (R) ou (S)-pseudo-*o-*bisphosphino-[2,2]-paracyclophane de formule générale VI 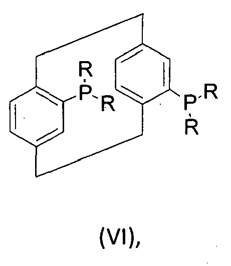
où R sont les mêmes ou différents substituants du groupe consistant des alkyles C₁₋₄, cycloalkyles C₃₋₈ et Ar,
et Ar est un aryle substitué ou non,
et, éventuellement, autre(s) component(s) nécessaire(s) pour formation du composé complexe.

2. Procédé selon la revendication 1, **caractérisé en ce que** le composé complexe, servant comme le catalyseur, contient un ligand (R) ou (S)-pseudo-*o*-bisphosphino-[2,2]-paracyclophane de formule générale VI, où R est un aryle substitué ou non.

3. Procédé selon les revendications 1 ou 2, **caractérisé en ce que** le composé complexe servant comme le catalyseur contient un ligand (R) ou (S)-pseudo-*o*-bisphosphino-[2,2]-paracyclophane de formule X

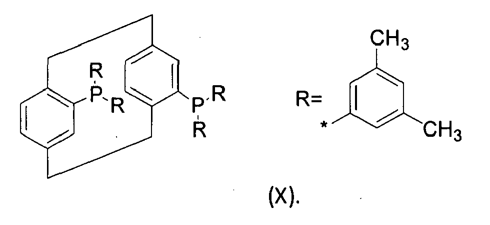

4. Procédé selon l'une quelconque des revendications 1-3, **caractérisé en ce que** le composé complexe servant comme le catalyseur contient un ligand (R)-pseudo-*o-*bisphosphino-[2,2]-paracyclophane de formule X.

5. Procédé selon l'une quelconque des revendications 1-4, **caractérisé en ce que** le composé complexe servant comme le catalyseur est préparé séparément ou in-situ.

6. Procédé selon l'une quelconque des revendications 1-5, **caractérisé en ce qu'**il est effectué dans une atmosphère contenant d'hydrogène gazeux.

7. Procédé selon l'une quelconque des revendications 1-6, **caractérisé en ce qu'**il est effectué an présence d'un sel d'ammonium.

8. Procédé selon la revendication 7, **caractérisé en ce que** le sel d'ammonium est choisi parmi le groupe contenant le chlorure d'ammonium, le phosphate d'ammonium, le formiate d'ammonium, l'acétate d'ammonium, l'oxalate d'ammonium, le carbonate d'ammonium, les sels d'ammonium des acides salicylique, citrique ou camphorique.

9. Procédé selon l'une quelconque des revendications 7 et 8, **caractérisé en ce que** le sel d'ammonium est choisi parmi le groupe contenant le sel d'ammonium de l'acide salicylique, le sel d'ammonium de l'acide formique et le sel d'ammonium de l'acide camphorique.

10. Procédé selon l'une quelconque des revendications 1-9, **caractérisé en ce qu'**il est effectué dans une atmosphère contenant d'hydrogène.

11. Procédé selon l'une quelconque des revendications 1-10, **caractérisé en ce qu'**il est effectué sous une pression d'hydrogène de 1-4 MPa.

12. Procédé selon l'une quelconque des revendications 1-11, **caractérisé en ce qu'**il est effectué à une température de 10-90 °C.

13. Procédé selon l'une quelconque des revendications 1-12, **caractérisé en ce qu'**il est effectué à une température de 40-70 °C.

14. Procédé selon l'une quelconque des revendications 1-13, **caractérisé en ce qu'**il est effectué dans des alcools C1-C6.

15. Procédé selon l'une quelconque des revendications 1-14, **caractérisé en ce qu'**il est effectué dans le 2,2,2-trifluoroéthanol.
